(19) Europäisches Patentamt
European Patent Office
Office européen des brevets

(11) Publication number: **0 210 665**
**B1**

(12) # EUROPEAN PATENT SPECIFICATION

(45) Date of publication of patent specification: **18.10.89**

(51) Int. Cl.$^4$: **C 07 J 1/00,** C 07 J 41/00, A 61 K 31/565

(21) Application number: **86110648.2**

(22) Date of filing: **01.08.86**

(54) **Steroids useful as anti-cancer, anti-obesity, anti-hyperglycemic, anti-autoimmune and anti-hypercholesterolemic agents.**

(30) Priority: **02.08.85 US 762584**

(43) Date of publication of application:
**04.02.87 Bulletin 87/06**

(45) Publication of the grant of the patent:
**18.10.89 Bulletin 89/42**

(84) Designated Contracting States:
**BE CH DE FR GB IT LI LU NL**

(56) References cited:
**EP-A-0 133 995**

(73) Proprietor: **Research Corporation**
**Suite 853, 25 Broadway**
**New York New York 10004 (US)**

(72) Inventor: **Schwartz, Arthur G.**
**220 Locust Street**
**Philadelphia Pennsylvania (US)**
Inventor: **Lewbart, Marvin Louis**
**546 East Street Andrews Drive**
**Media Pennsylvania (US)**
Inventor: **Williams, John R.**
**824 North Woodbine Avenue**
**Penn Valley, Pennsylvania (US)**
Inventor: **Abou-Gharbia, Magid**
**2106 Weatherton Drive**
**Brandywood, Wilmington, Delaware (US)**

(74) Representative: **Lehn, Werner, Dipl.-Ing. et al**
**Hoffmann, Eitle & Partner Patentanwälte**
**Arabellastrasse 4**
**D-8000 München 81 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 210 665 B1

Courier Press, Leamington Spa, England.

**Description**

The invention described herein was made in the course of work under a grant or award sponsored in part by the National Institutes of Health.

This invention relates to novel steroids and more particularly to androsterone derivatives useful as anti-cancer, anti-obesity, anti-diabetic and hypolipidemic agents.

Dehydroepiandrosterone (DHEA) and DHEA-sulfate are major adrenal secretory products in humans. The plasma concentration of DHEA-sulfate, which, next to cholesterol, is the most abundant steroid in humans, undergoes the most marked age-related decline of any known steroid.

Although, DHEA-sulfate is the main precursor of placental estrogen and may be converted into active androgens in peripheral tissue, there is no obvious biological role for either DHEA or DHEA-sulfate in the normal individual. Several retrospective and prospective studies suggest that women with sub-normal levels of these steroids may be predisposed to develop breast cancer. For example, see Brownsey et al, "Plasma dehydroepiandrosterone sulfate levels in patients with benign and malignant breast disease", Eur. J. Cancer, *8,* 131—137 (1972); Bulbrook et al, "Relation between urinary androgen and corticoid excretion and subsequent breast cancer", Lancet, *2,* 395—398 (1971); Rose et al, "Plasma dehydroepiandrosterone sulfate, androstenedione and cortisol, and urinary free cortisol excretion in breast cancer", Eur. J. Cancer, *13,* 43—47 (1977); Wang et al, "Studies on the sulfate esters of dehydroepiandrosterone and androsterone in the blood of women with breast cancer", Eur. J. Cancer, *10,* 477—482 (1974); and Zumoff et al, "Abnormal 24-hr mean plasma concentrations of dehydroisoandrosterone and dehydroisoandrosterone sulfate in women with primary operable breast Cancer", Cancer Research, *41,* 3360—3363, September 1981.

It has also been established that DHEA is a potent non-competitive inhibitor of mammalian glucose-6-phosphate dehydrogenase (G6PDH). For example, see Oertel et al, "The effects of steroids on glucose-6-phosphate dehydrogenase", J. Steroid Biochem., *3,* 493—496 (1972) and Marks et al, "Inhibition of mammalian glucose-6-phosphate dehydrogenase by steroids", Proc Nat'l. Acad. Sci, USA, *46,* 447—452 (1960). Moreover, Yen et al, "Prevention of obesity in $A^{vy}$/a mice by dehydroepiandrosterone", Lipids, *12,* 409—413 (1977), reported that long-term administration of DHEA to VY-$A^{vy}$/a mice prevented the development of obesity without suppressing appetite.

Furthermore, it is also known that the long-term treatment of C3H mice with DHEA, in addition to reducing weight gain without suppressing appetite, markedly inhibits spontaneous breast cancer development and may delay the rate of aging. It has been observed that DHEA antagonizes the capacity of the tumor promoter, 12-O-tetradecanoylphorbol-13-acetate, to stimulate $^3H$-thymidine incorporation in mouse epidermis and in a cultured rat kidney epithelial cell line. See, Schwartz, "Inhibition of spontaneous breast cancer formation in female C3H-$A^{vy}$/a mice by long-term treatment with dehydroepiandrosterone", Cancer Res., *39,* 1129—1132 (1979); and Schwartz et al, "Dehydroepiandrosterone: an anti-obesity and anti-carcinogenic agent", Nut. Cancer *3,* 46—53 (1981).

Ben-David et al, "Anti-hypercholesterolemic effect of dehydroepiandrosterone in rats", Proc. Soc. Expt. Biol. Med., *125,* 1136—1140 (1967) have observed that DHEA treatment has an anti-hypercholesterolemic effect in mice, while Coleman et al (Diabetes *31,* 830, 1982) report that administration of DHEA produces a marked hypoglycemic effect in C57BL/KsJ-db/db mice. The latter authors suggest that the therapeutic effect of DHEA might result from its metabolism to estrogens.

It is further known that DHEA and 16α-bromoepiandrosterone are inhibitors of Eptstein-Barr virus-induced transformation of human lymphocytes and that 16α-bromo-epiandrosterone is a more potent inhibitor of mammalian G6PDH than DHEA. See, Schwartz et al Carcinogensis, Vol. 2 No. 7, 683—686 (1981).

While DHEA has been found effective in the afore-described manners, there is, however, evidence of an estrogenic effect after prolonged administration. DHEA is not an estrogen *per se* but is well known to be convertible into estrogens. In addition, the therapeutic dose of DHEA is rather high. It would therefore be highly desirable to provide steroids, which while having the same afore-described advantages of DHEA are more potent and do not produce an estrogenic effect.

EP—A—133 995 describes steroids, which are useful as anti-cancer, anti-obesity, anti-hyperglycemic, anti-autoimmune and anti-hypercholesterolemic agents. Among the compounds described are compounds having the formula

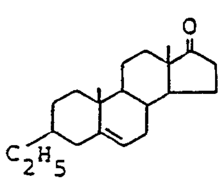

13

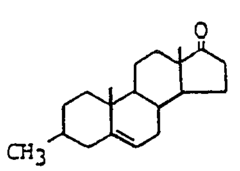

16

and

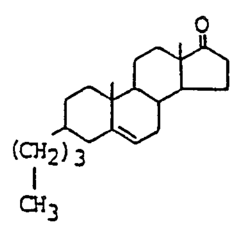

17

Said compounds provide an inhibition of TPA (tumor promoter) stimulation of mouse epidermal DNA.

The present invention provides novel steroids exhibiting significant and desirable pharmacological properties, and are particularly useful as cancer preventive agents.

The above-identified steroids are additionally useful as anti-obesity agents, anti-hyperglycemic agents, anti-aging agents, and anti-hypercholesterolemic agents.

This invention further provides steroids useful as anti-cancer, anti-obesity, anti-hyperglycemic, anti-aging and anti-hypercholesterolemic agents, which do not evidence estrogenic effects.

The present invention comprises compounds having the formula

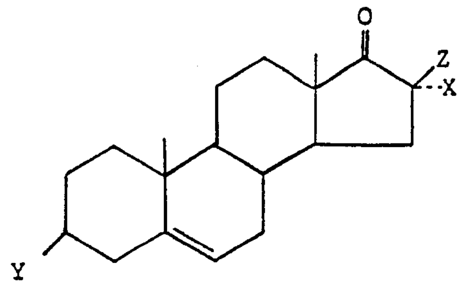

wherein

Y is $C_{1-5}$ alkyl; and

X and Z are independently hydrogen, halogen, $C_{1-5}$ alkyl or hydroxy, or X and Z taken together are hydroxyimino with the proviso that when Y is methyl and Z is hydrogen, X is other than methyl or hydroxy and with the further proviso that X and Z are not both hydrogen and that said compound is not 3β-methyl-16 α-ethyl-5-androsten-17-one or 16 α-bromo-3β-methyl-5-androsten-17-one.

Preferred compounds of the present invention are of the formula:

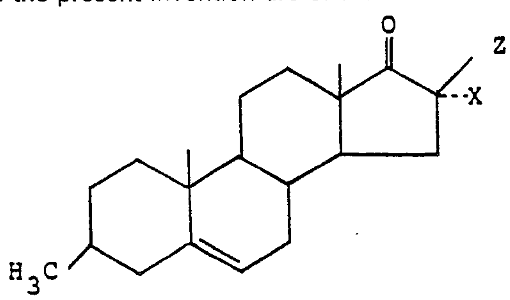

wherein

Z is hydrogen or $C_{1-5}$ alkyl;

X is halogen or $C_{1-5}$ alkyl; and

X and Z taken together are hydroxyimino with the proviso that when Z is hydrogen, X is other than methyl.

In accordance with the present invention, it has been surprisingly discovered that steroids having a certain structure, described hereinafter in more detail, are characterized with significant pharmacological properties without toxic or undesirable estrogenic effects. That is, it has been quite unexpectedly discovered that the steroids of the present invention are useful as cancer preventive, anti-obesity, anti-diabetic, anti-aging and anti-hyper cholesterolemic agents, but unlike DHEA are more potent and exhibit very little or no estrogenic effects.

In the present invention the term alkyl comprises straight or branched chain, alkyl groups with up to 5 carbon atoms. Examples include methyl, ethyl, propyl, isopropyl, butyl, isobutyl, pentyl and amyl. The most preferred alkyl group is methyl.

The halo atoms are preferably Br, F, or Cl, especially F or Br.

The various substituents are designated as being in the α-position by means of a broken line (---) joining the substituent to the steroid nucleus, the substituents are designated as being in the β-position by means of a solid line (—) joining the substituent to the steroid nucleus and in those cases in which the substituent may be either in the α- or β- position the substituents are indicated as being joined to the steroid nucleus by a broken line and a solid line placed side to side. Furthermore, in accordance with I.U.P.A.C. nomenclature, the carbon atoms of the steroids of the present invention are numbered as follows and the steroids have the designated I.U.P.A.C. stereochemistry:

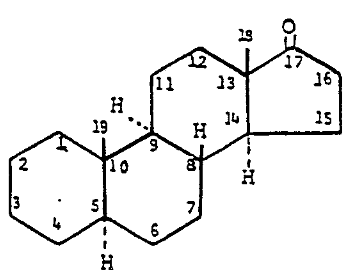

Specific illustrative compounds of structural Formula I and useful in accordance with the present invention include:

16α-fluoro-3β-methylandrost-5-en-17-one;

16α-bromo-3β-methylandrost-5-en-17-one;

16α-hydroxyimino-3β-methylandrost-5-en-17-one;

16α-3fluoro-3β,16β-dimethyl-5-androsten-17-one.

The steroids of the present invention may be prepared in accordance with conventional organic syntheses. The following procedures are illustrative of some procedures which may be utilized to prepare the steroids included herein:

Carbon 3-Alkylations

The schematic for carbon 3-alkylations are shown figuratively in scheme 1 below.

Synthesis of dehydroepiandrosterone with a methyl group replacing the hydroxyl group at carbon-3 is shown below in scheme 1. The methyl configuration at carbon-3 is β, as determined by X-ray analysis. 3β-Hydroxyandrost-5-en-17-one (*10*) was iodinated at carbon-3 with catechol phosphochloridate followed by iodine. 3β-Iodoandrost-5-en-17-one (*11*) was ketalized then alkylated with a mixture of methyl lithium and cuprous cyanide, in tetrahydrofuran to yield 3β-methylandrost-5-en-17-ethylene ketal (*13*). Hydrolysis of the ketal afforded 3β-methylandrost-5-en-17-one (*14*).

Scheme 1

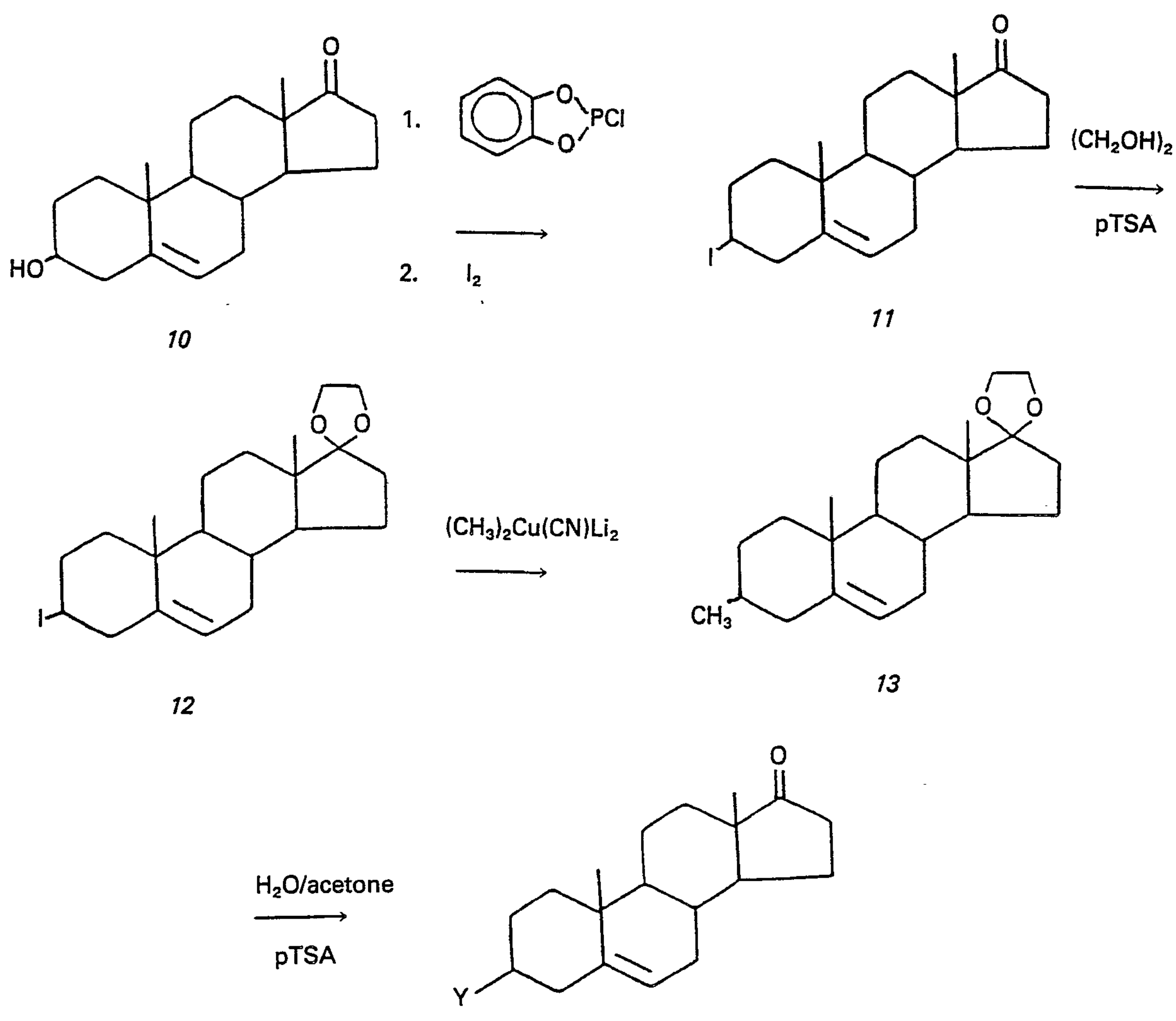


10 11 12 13 14

More specifically, 3β-iodoandrost-5-en-17-one (*11*) (11.83 g, 29.7 mmol) ethylene glycol (20 ml) and p-toluene sulfonic acid (200 mg) in benzene (250 ml) were refluxed under a Dean-Stark trap for 72 h. The solution was washed with saturated sodium bicarbonate, water, then dried over magnesium sulfate. Evaporation and recrystallization from ether afforded 11.5 g (87.3%) of 3β-iodoandrost-5-en-17-one 17-ethyleneketal (*12*): mp 140—141°C.

IR(KBr) 3010, 2940, 1470, 1425, 1375 $cm^{-1}$; $^1H$ NMR ($CDCl_3$) δ 5.44 (brd, J=6Hz, 1H, H—6) 3.91 (s, 4H, ketal) 1.07 (s, 3H, C—19 Me) .88 (s, 3H, C—18 Me); MS (m/e) 442 ($M^+$, 1), 380 (35), 315 (57), 253 (67), 227 (11), 105 (24), 99 (100), 91 (35), 55 (27), 41 (33).

Cuprous cyanide (4.465 g, 49.9 mmol) was placed in a dry 500 ml 3 neck round bottom flask equipped with a magnetic stirrer. The system was flushed with $N_2$ and dry THF (30 ml) was added. The suspension was cooled to −78°C and MeLi 1.5 *M* (66.5 ml, 99.8 mmol) was added via syringe. The solution was allowed to warm to 0°C for 5 min, which resulted in a clear tan solution.

After recooling to −78°C, the 3β-iodo-17-ketal (*3*) (7.35 g 16.6 mmol) in 40 ml dry tetrahydrofuran was added via a syringe and the solution allowed to warm to room temperature and stirred for 18 hrs under $N_2$. The solution was extracted with 100 ml of 90% saturated $NH_4Cl$/10% conc. $NH_4OH$. The organic layer was separated, dried over $MgSO_4$ and evaporated to give 6.69 g of crude product. Chromatography on flash silica (240 g) and elution with 1% $Et_2O$/99% hexane gave 6.41 g of colorless crystals. Recrystallization from methanol (200 ml) gave 3β-methylandrost-5-en-17-one 17-ethyleneketal, mp 121—122°C.

Anal. Calc. C 80.06 H 10.38.
Found C 80.12 H 10.55.

IR (KBr) 3010, 2930, 1450, 1430, 1370; $^1H$ NMR ($CDCl_3$) δ 5.33 (brd, J=6Hz, 1H, H—6), 3.90 (s, 4H, ketal) 1.03 (s, 3H, C—19 Me) .91 (s, 3H, C—18 Me) .97 (d, 3H, C—3 Me); MS (m/e) 330 ($M^+$, 16), 316 (7), 268 (29), 253 (22), 239 (9), 99 (100), 91 (22), 55 (27), 41 (22).

The 3β-methylandrost-5-en-17-one 17-ethylene-ketal (*13*) (2.20 g 6.7 mmol) was dissolved in acetone (100 ml). p-Toluene sulfonic acid (100 mg) and $H_2O$ (20 ml) were added and the solution refluxed for 2 h. The solution was evaporated, taken up in ether (30 ml), washed with saturated $NaHCO_3$, $H_2O$, then dried over $MgSO_4$. The solution was filtered and evaporated to give a colorless solid which was recrystallized from methanol to give 3β-methylandrost-5-en-17-one (*14*) colorless plates 1.17 g (61%), mp. 148—150°C;

IR (KBr) 3010, 2910, 1740, 1455, 1430, 1366; $H^1$ NMR ($CDCl_3$) δ 5.41 (brd, J=6Hz, 1H, H—6) 1.11 (s, 3H, C—19 Me) 0.99 (s, 3H, C—18 Me) 1.07 (d, 3H, C—3 Me); MS (m/e) 286 ($M^+$, 58) 271 (51), 229 (31), 159 (36), 105 (72), 91 (95), 79 (89), 55 (9), 41 (100).

Anal. Calc. C 83.85 H 10.55.
Found C 83.66 H 10.65.

Using the above procedure and substituting $(Y)_2Cu(CN)Li_2$ for $(CH_3)_2Cu(CN)Li_2$, other 3-alkyl derivatives can be prepared.

Alkylation at Carbon-16

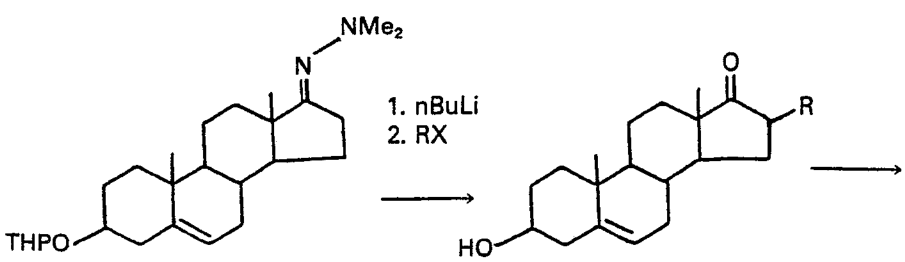


*1*
3. CuCl. aq THF
*2*

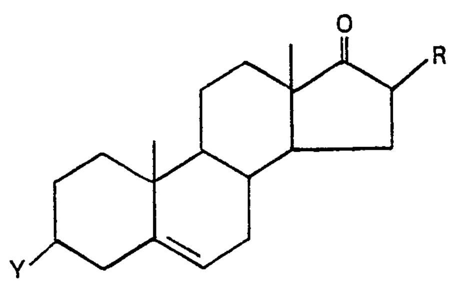

*3*

Alkylation of the 17-ketodimethylhydrazone of DHEA 3-tetrahydropyranyl ether using n-butyl lithium as the base followed by an alkyl halide RX, afforded the 16α-alkylated steroid. Hydrazone cleavage with cuprous chloride in aqueous tetrahydrofuran led to regeneration of the C—17 ketone and concomitant cleavage of the tetrahydropyranyl ether resulting in the 16α-alkyl-3β-hydroxy-androst-5-en-17-one *2*.

The final product *3* was formed by following the procedure outlined in the section entitled, Carbon-3-Alkylations, Supra.

The following procedures illustrate hydroxylation at carbon-16.

Hydroxylation at Carbon-16

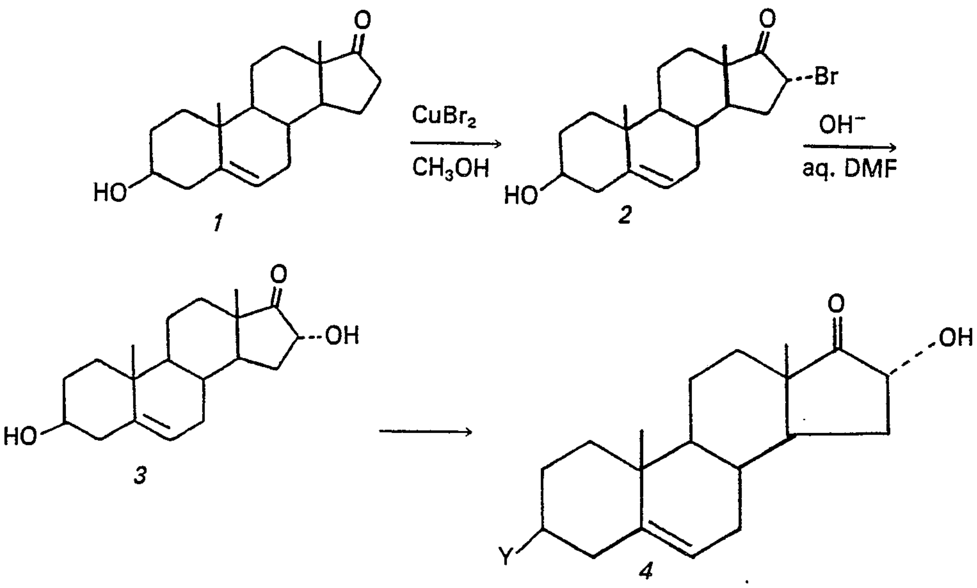


1 2 3 4

Bromination of DHEA (1) with cupric bromide in methanol yields 16α-bromo-DHEA, *2*. The bromo ketone *2* was treated with 75% aqueous pyridine in an oxygen atmosphere with 1.8 equivalents of NaOH. The reaction mixture was added to an ice/$H_2O$ mixture containing an equivalent amount of HCl. The product was extracted with $CH_2Cl_2$, and washed with $H_2O$ and dried over $Na_2SO_4$. The product crystallized out solution and the crystals and mother liquor were subjected to reverse phase HPLC, using acetonitrile/$H_2O$ as eluent. The melting point of the colorless solid was 157—160°C. Similarly, 3-β-methyl-16α-hydroxyandrost-5-en-17-one may be prepared by hydroxylation of 3-β-methylandrost-5-en-17-one using this procedure to introduce the 16α-hydroxy group.

Following the procedures outlined in the Section entitled Carbon-3-Alkylations, Supra, 4, can be prepared from *3*.

The following procedures are representative of procedures for halogenation at carbon-3 or 16.

Halogenation at Carbon-16

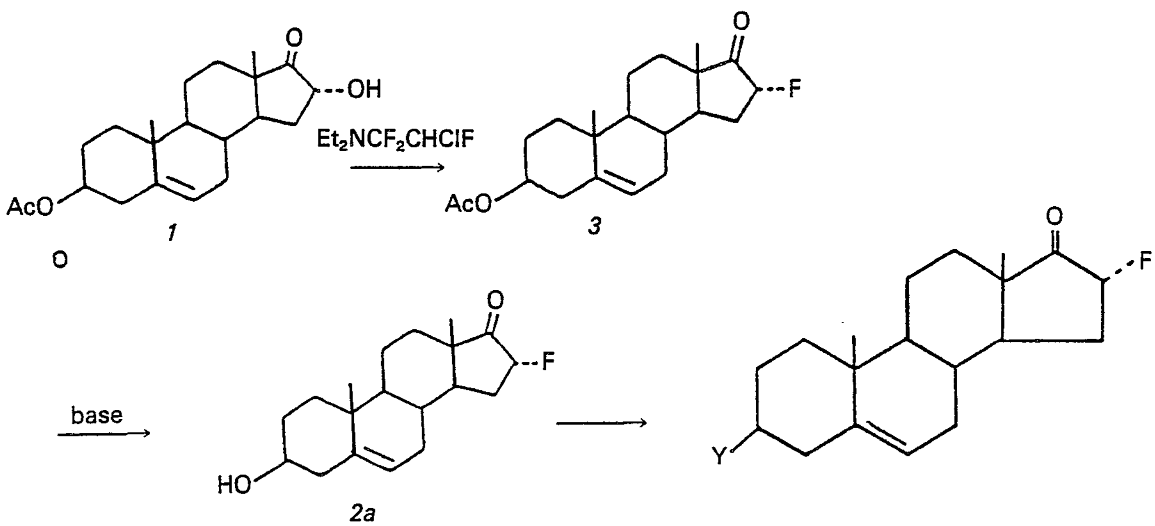


1 3 2a

Reaction of 3β,16α-dihydroxyandrost-5-en-17-one 3β-acetate *1* with diethyl (2-chloro-1,1,2-trifluoroethyl)amine affords 16α-fluoro-3β-hydroxyandrost-5-en-17-one 3-acetate *3*. Hydrolysis of the ester with base yields 16α-fluoro-3β-hydroxyandrost-5-en-17-one, *2a*.

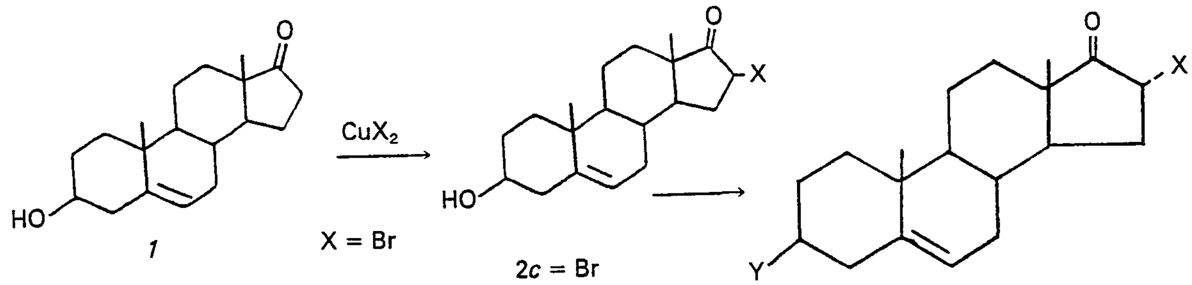


1    2c = Br

Reaction of 3β-hydroxyandrost-5-en-17-one *1* with cupric bromide yields 16α-bromo-3β-hydroxyandrost-5-en-17-one, *2c*[1].

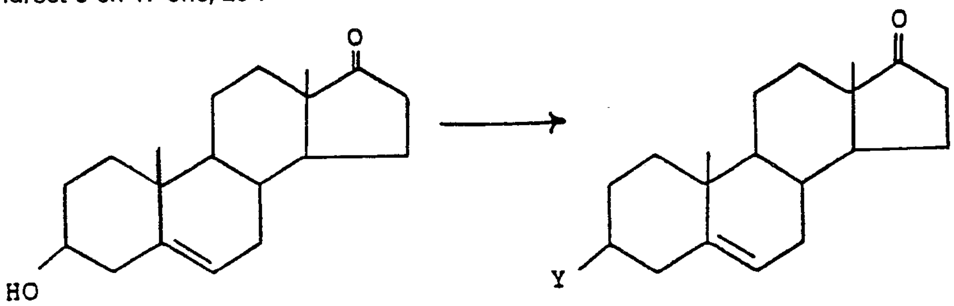

1    2

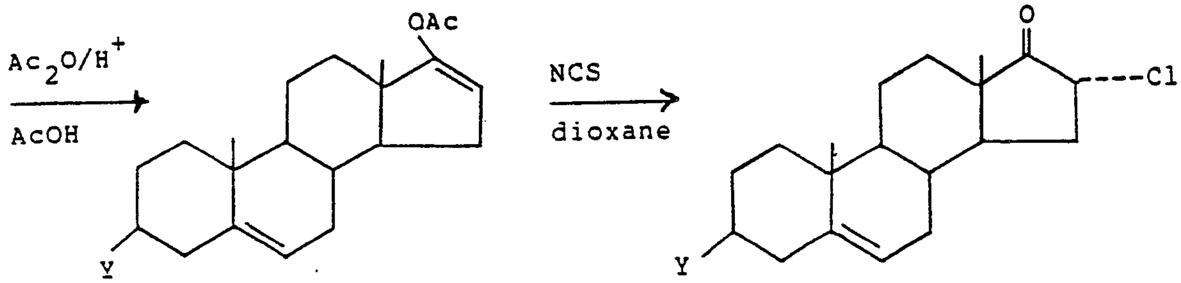


3    4

The 16 chloro derivative can be prepared by converting DHEA *1* to the 3-alkyl derivative *2* in accordance with the procedure outlined in the section entitled Carbon-3-alkylations, *supra*. The reaction of *2* with acetic anhydride affords the enol acetate, *3*. The reaction of 3 with N-chlorosuccinimide (NCS) yields the 3-alkyl-16α-chloro-5-androsten-17-one.

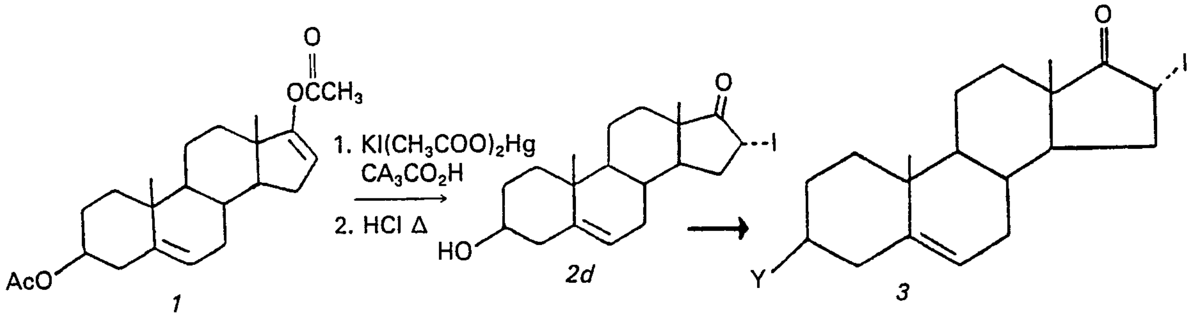


1    2d    3

Reaction of 3β,17-dihydroxyandrosta-5,16-diene 17-acetate *1* with mercuric acetate followed by treatment with potassium iodide yielded the C—16α iodide which hydrolyses with acid to yield 3β-hydroxy-16α-iodoandrost-5-en-17-one, *2d*. Reaction of *2d* with silver fluoride yields 3β-hydroxy-16α-fluoroandrost-5-en-17-one, *2a*.

In addition, the reaction of *2c* with NaI/acetone overnight results in a mixture of 16α-I-3β-hydroxyandrost-5-en-17-one.

Alternatively, *2d* can be formed from the reaction of *1* with N-iodo-succinimide.

---

[1]E. R. Glazier J. Org. Chem. 1962, *27*, 4397.

Using the procedures discussed in the section entitled *Carbon 3-Alkylations, Supra,* 2a, 2b, and 2d can be alkylated in the 3-position of the steroid to form the final products, *3.*

Halogenation at Carbon-3

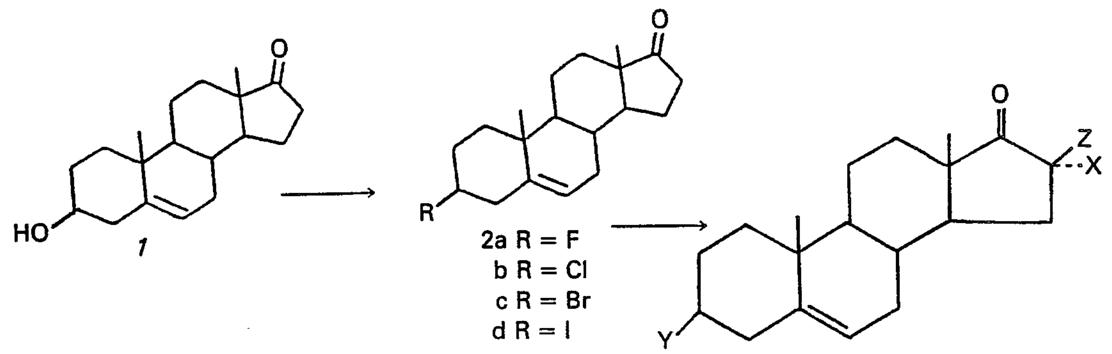

*1*

2a R = F
b R = Cl
c R = Br
d R = I

*3*

Reaction of 3β-hydroxyandrost-5-en-17-one *1* with diethyl (2-chloro-1,1,2-trifluoroethyl) amine yields 3β-fluoroandrost-5-en-17-one *1*. Reaction of *1* with thionyl chloride yields 3β-chloroandrost-5-en-17-one, *2b.* Reaction of *1* with phosphorus tribromide yields 3β-bromoandrost-5-en-17-one, *2c.* Reaction of *1* with o-phenylene phosphochloridite followed by iodine yields 3β-iodoandrost-5-en-17-one *2d.*

Using the appropriate procedures discussed in the previous sections, *3* can be formed from *2a,* 2b, 2c, or 2d.

In the formation of the compounds of the present invention, the carbon 3-alkylation can be formed prior to or subsequent to the substitution at the 16-position.

The following Examples further illustrate the invention:

Example I

16-Hydroxyimino-3β-methylandrost-5-en-17-one

3β-methylandrost-5-en-17-one, which was prepared according to the procedure described hereinabove, was dissolved in t-butanol, and metallic potassium was added. Isoamyl nitrite was added dropwise under nitrogen and the mixture was refluxed for 15 minutes. The dark red reaction mixture was quenched in an ice bath. The crude product was filtered off and purified by reverse phase HPLC, using an octadecylsilane column and acetonitrile/$H_2O$ as the eluent. A light yellow solid was isolated, m.p. 168—170°C.

Example II

3β,16β-dimethylandrost-5-en-17-one

To a solution of 16β-methyl-3β-hydroxy-5,16-pregnadien-20-one was added toluene, ethylene glycol, and p-toluene-sulfonic acid. The resulting solution was refluxed overnight forming the 20-ketal. The procedure for this ketalization step is described in *JACS, 76,* 5674 (1954). Tosyl chloride in pyridine was added to the above product to form the 3β-tosylate derivative. The 3β-tosylate was refluxed overnight with 10% NaI/acetone to form the 3β-iodo-16-methyl-5,16-pregnadien-20-one-ethylene ketal. This product was methylated with lithium dimethylcuprate in ether and tetrahydrofuran at −78°C under a nitrogen atmosphere to form the 3β,16-dimethyl-5,16-pregnadien-20-one ethylene ketal. This product was deketalized by refluxing in the presence of acetone/p-toluenesulfonic acid. The resulting 3β,16-dimethyl,5,16-pregnadien-20-one was converted to the C—20-oxime by refluxing in ethanol and pyridine with an excess of hydroxylamine hydrochloride. This product was subjected to a Beckmann rearrangement in the presence of p-acetamidobenzenesulfonyl chloride pyridine according to the procedure by Rosenkranz, et al in *J. Org. Chem., 21,* 520—522 (1956). The product, 17-acetamido-3β,16-dimethyl-5,16-androstadiene was refluxed in tetrahydrofuran/hydrochloride acid solution. 3β,16β-dimethylandrost-en-17-one was formed, separated from impurities and purified by normal phase HPLC using a 1 in × 25 cm silica gel preparative column at a flow rate of 30 ml/min and using ethyl acetate/hexane (in a gradient ranging from 0 to 20%) as the eluent. The products were recrystallized from methanol and characterized by NMR and IR. Colorless crystals of the products were isolated: 3β,16β-dimethyl-5-androsten-17-one m.p. 86.5—88.5°C.

Similarly, using the appropriate starting materials, the following components can also be prepared:

3β,16β-diethyl-5-androsten-17-one

3β-methyl-16β-ethyl-5-androsten-17-one.

Example III.

3β-methyl-16α-fluoro-androst-5-en-17-one

The 17-acetamido-3β,16α-dimethyl,5,16-pregadien which was formed above was treated with

perchloryl fluoride ($FClO_3$) to form the 16α-F-3β-methyl-17-acetamido-5-androsten. Hydrolytic cleavage in aqueous THF containing HCl afforded the final product.

Example IV

16α-fluoro-3β,16β-dimethyl-5-androst-en-17-one

The procedure is identical to the formation of 3β-methyl-16α-fluoro-androst-5-en-17-one except that the starting material is 3β,16-dimethyl-3β-hydroxy-5,16-pregadien-20-one. The final product which was purified by normal phase HPLC using ethylacetate-hexane as eluent, and recrystallized from methanol, formed a colorless crystal which melted at 129—130°C.

Example V

16α-bromo-3β-methyl-5-androst-en-17-one

3β-methyl-5-androst-en-17-one was refluxed with cupric bromide in methanol overnight. The reaction mixture was dumped in water and the crystals were collected by filtration.

Example VI

16β-fluoro-3β-methyl-5-androst-en-17-one

The 16α-bromo derivative that was formed above was refluxed in isopropanol and toluene containing AgF to form the final product.

Example VII

16α-iodo-3β-methyl-5-androst-en-17-one

16β-iodo-3β-methyl-5-androst-en-17-one

The 16α-bromo derivative that was formed above was refluxed with sodium iodine in acetone overnight. The mixture of the 16α-iodo and the 16β-iodo derivatives were formed which were separated by normal phase HPLC, using silica gel as adsorbent in a column 2.54 cm (1 in) × 25 cm, using ethyl acetate:hexane (gradient from 0 → 20%) as the eluent.

Inhibition of G6PDH

The compounds listed below are screened as inhibitors of purified bovine adrenal G6PDH activity as one predictor of cancer preventive action. The assay for testing the inhibition of purified bovine adrenal G6PDH is according to the procedure by Oertell, G. W. and Rebelein, I. in Biochem. Biophys. Acta, *184,* 459—460 (1969). The results are given below in Table I:

G6PDH INHIBITION TEST

TABLE I

| Compound | No. | Conc. | Per Cent Inhibition |
|---|---|---|---|
| DHEA | *1* | 10 µm | 51, 52 |
| | | 1 µm | 20, 23 |
| 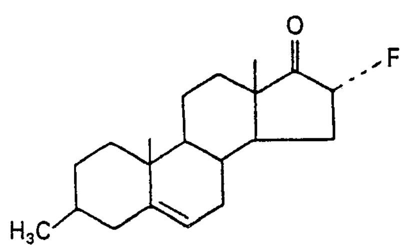 | *20* | 10 µm | 46, 52 |
| | | 1 µm | 55, 56 |
| DHEA | *1* | 10 µm | 53, 57 |
| | | 1 µm | 17, 17 |
| 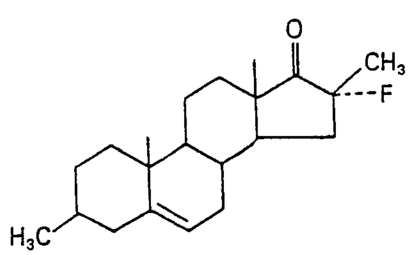 | *22* | 10 µm | 65, 71 |
| | | 1 µm | 53, 63 |

G6PDH INHIBITION TEST

TABLE I cont'd

| Compound | No. | Conc. | Per Cent Inhibition |
|---|---|---|---|
| DHEA | *1* | 10 µm | 53 |
| | | 1 µm | 19 |
| 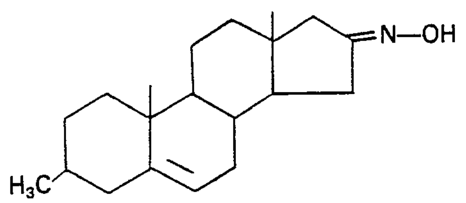 | *23* | 10 µm | 75 |
| | | 1 µm | 25 |

BACKGROUND INFORMATION ON ACTIONS OF DMBA AND TPA

Skin tumors can be induced in the mouse either by weekly application of a carcinogen such as 7,12-dimethylbenzylanthracene (DMBA), or alternatively, by a single sub-threshold dose of the carcinogen followed by twice weekly applications of the tumor promoter tetradecanoylphorbol-13-acetate (TPA). In order to exert its carcinogenic effect, DMBA must be metabolized by an NADPH-dependent mixed-function oxidase to chemically reactive intermediates which bind covalently to DNA and produce mutations leading to malignant transformation. Dehydroepiandrosterone (DHEA) and 3β-methylandrost-5-en-17-one inhibit 7,12-dimethylbenz(a)anthracene (DMBA)-initiated and 12-O-tetradecanoylphorbol-13-acetate (TPA)-promoted skin papilloma formation in mice, *Carcinogenesis*, 5, 464—466 and DHEA inhibits the rate of binding of topically applied $^3$H-DMBA to A/J mouse skin DNA (Table 4). The potent androgen, testosterone, is without inhibitory effect. This effect of DHEA very probably is a result of the inhibition of G6PDH and lowering of the intracellular pool of NADPH, which is a co-factor for the mixed-function oxidase activation of DMBA. Topical DHEA or 3β-methylandrost-5-en-17-one application also inhibits DMBA produced papillomas and carcinomas in the complete carcinogenesis model (Pashko, L. L. Hard, G. C.; Rovito, R. J. ; Williams, J. R.; Sobel, E. L.; and Schwartz, A. G. (1985). Inhibition of 7,12-dimethylbenz(a)anthracene induced skin papillomas and carcinomas by dehydroepiandrosterone and 3β-methylandrost-5-en-17-one in mice, *Cancer Res., 45,* 164—166).

Tumor promoters, such as TPA, stimulate hyperplasia and DNA synthesis when applied to the skin, and it is believed that this stimulation is an important step in the enhancement of tumorigenesis. This stimulation of epidermal DNA synthesis rate by TPA can be demonstrated by an enhanced rate of $^3$H-thymidine incorporation in mouse epidermis 20 hours after TPA application. Again, topical DHEA treatment abolishes this stimulation (Table 5).

The inhibition of the TPA stimulation of epidermal $^3$H-thymidine incorporation by DHEA may also result from G6PDH inhibition. The pentose-phosphate pathway provides both ribose-phosphate for ribonucleotide synthesis as well as NADPH which is needed both for the reduction of folic acid to tetrahydrofolic acid (required for ribonucleotide and thymidylate synthesis) as well as for the activity of ribonucleotide reductase. DHEA, over a range of $10^{-5}$M to $10^{-4}$M, slows the growth of many different cell lines in culture. One HeLa cell strain, TCRC-2, is particularly sensitive to DHEA-induced growth inhibition. This growth inhibition can be almost completely overcome by adding to the culture medium a mixture of the deoxynucleosides of adenine, guanine, cytosine, and thymine, which is consistent with the hypothesis that DHEA inhibits cell growth through G6PDH inhibition (Dworkin, C. R., Gorman, S. D., Pashko, L. L., Cristofallo, V. J. and Schwartz, A. G. (1986). Inhibition of growth of HeLa and WI-38 cells by dehydroepiandrosterone and its reversal by ribo- and deoxyribonucleosides, *Life Sci., 38,* 1451—1457).

FOOD RESTRICTION AND CANCER PREVENTION

It has been known for 45 years that reducing the food intake of laboratory mice inhibits the development of a broad spectrum of spontaneous and chemically induced tumors (Tannenbaum, A. (1940); The Inhibition and Growth of Tumors. Introduction. I. Effects of Underfeeding, *Am. J. Cancer, 38,* 335—350), but the mechanism of this effect is not clear. It appears that food restriction of mice for two weeks inhibits both the binding of $^3$H-DMBA to skin DNA as well as the TPA stimulation of epidermal $^3$H-thymidine incorporation (Tables 2 and 3) to a degree comparable to that observed with an application of 400 µg of DHEA. Both these effects of food restriction very likely result from a depression in G6PDH activity (Table 5). Thus inhibition of G6PDH activity may be an important component in the cancer preventive effects of both food restriction and DHEA treatment.

Administration of DHEA at a daily dose of approximately 400 mg/kg in long-term experiments has been shown to inhibit the development of breast, lung, and colon tumors. This dose of DHEA, when administered repeatedly over a period of a few weeks, also produces an anti-weight effect. However, a

single administration of DHEA at 400 mg/kg to mice does not inhibit $^3$H-DMBA binding to skin DNA and does not inhibit the TPA stimulation in epidermal $^3$H-thymidine incorporation to a degree comparable to that produced by either food restriction or a topical application of 400 µg of DHEA. (Table 7 vs. Tables 2, 3 and 4). However, treatment of mice for four weeks with 400 mg/kg of DHEA does inhibit $^3$H-DMBA binding to skin DNA, but this regimen of DHEA treatment also produces an anti-weight effect, which is due to both a reduction in food intake and to a decrease in the efficiency of food utilization. Thus the cancer preventive effect of DHEA may result indirectly from its anti-weight action rather than from a direct effect of DHEA or target cells.

However compound *20*, when administered orally to mice, inhibits $^3$H-DMBA binding to skin DNA and TPA stimulation in $^3$H-thymidine incorporation to a degree comparable to that produced by food restriction at doses well below 400 mg/kg, whereas DHEA is inactive. (Tables 6, 7, 8, and 9). At these dosages the new compounds do not produce an anti-weight effect. Therefore, the cancer preventive activities of the present compounds are more potent than the cancer preventive activity of DHEA, and in addition, the cancer preventive activity of the present new steroids has been dissociated from the anti-obesity effect.

TABLE 2

Effect of Steroid Treatment or Two Weeks of Food Restriction on ($^3$H) DMBA Binding to Skin DNA

| Treatment | Specific Activity (cpm/µg DNA) |
|---|---|
| *Ad libitum* fed | 116 ± 5.2 |
| *Ad libitum* fed plus DHEA | 66 ± 13 |
| *Ad libitum* fed plus testosterone | 164 ± 8.4 |
| Food restricted (two weeks) | 57 ± 14 |

Binding of [$^3$H]DMBA to mouse skin DNA was determined as described in Pashko, L. L., and Schwartz, A. G. (1983), Effect of food restriction, dehydroepiandrosterone, or obesity on the binding of $^3$H-7,12-dimethylobenz(a)-anthracene to mouse skin DNA, *J. Gerontol., 38,* 8—12. Values are mean ± SD for 3 individual determinations, with pooled tissue from 2 mice used for each determination. DHEA or testosterone (400 µg in 0.2 ml acetone) was applied to the skin one hour before [$^3$H]DMBA. The mean weight of the food restricted mice was 18.5 ± 1.0 g, n=6, of the *ad libitum* fed, 27.4 ± 1.0 g, n=6, of the *ad libitum* fed treated with DHEA, 28.2 ± 0.9 g, n=6, and of the *ad libitum* fed treated with testosterone, 28.3 ± 0.6 g, n=6, followed two weeks of feeding. The average food consumed was, in g/mouse/day, 2.2, 3.8, 3.8 and 4.0 for the food restricted, *ad libitum* fed, *ad libitum* fed plus DHEA, and *ad libitum* fed plus testosterone groups, respectively.

*Significantly less than *ad libitum* fed mice, $p < 0.01$;

**Significantly greater than *ad libitum* fed mice, $< 0.01$.

TABLE 3

Inhibition of TPA Stimulation of $^3$H-Thymidine incorporation in Epidermis by DHEA

| Treatment | Specific Activity (cpm/µg DNA) |
|---|---|
| No steroid | 66 ± 1.8 |
| No steroid plus TPA | 174 ± 35 |
| TPA plus DHEA (100 µg) | 52 ± 5.8 |
| TPA plus DHEA (400 µg) | 22 ± 6.5 |
| TPA plus testosterone (100 µg) | 128 ± 13 |
| TPA plus testosterone (400 µg) | 142 ± 5.9 |

Incorporation of $^3$H-thymidine into A/J mouse epidermal DNA was determined as described in Pashko, L. L., Schwartz, A. G., Abou-Gharbia, M. and Swern, D. (1981), Inhibition of DNA synthesis in mouse epidermis and breast epithelium by dehydropiandrosterone and related steroids, *Carcinogenesis, 2,* 717—721. Values are mean ± SD for 3 separately treated mice in each group 20 hours after TPA application. DHEA or testosterone was added topically in 0.2 ml acetone one hour before TPA addition.

TABLE 4

Effect of Two Weeks of Food Restriction on TPA Stimulation of Epidermal $^3$H-Thymidine Incorporation

| Treatment | Specific Activity (cpm/μg DNA) |
|---|---|
| *Ad libitum* fed | 54 ± 0.8 |
| *Ad libitum* fed plus TPA | 193 ± 25 |
| Food restricted (two weeks) plus TPA | 34 ± 6.8 |

Incorporation of $^3$H-thymidine into A/J mouse epidermal DNA was determined as described in Table 3. Values are mean ± SD for 3 separately treated mice in each group. The means weight of the food restricted mice was 18.3 ± .6 g n=3, and of the *ad libitum* fed was 26.7 ± 1.4, n=6, following two weeks of feeding. The average food consumed was 2.4 g/mouse/day for food restricted and 4.9 g/mouse/day for *ad libitum* fed mice.

TABLE 5

Effect of Two Weeks of Food Restriction on Epidermal G6PDH Activity

| Treatment | Specific Activity (nmoles NADPH/mg protein min) |
|---|---|
| *Ad libitum* fed | 43.4 ± 6.0 |
| Food restricted (two weeks) | 18.1 ± 5.1 |

Epidermal G6PDH activity was determined as described in Ziboh, V. A., Dreize, M. A., and Hsia, S. L. (1970), Inhibition of lipid synthesis and glucose-6-phosphate dehydrogenase in rat skin by dehydroepiandrosterone, *J. Lipid Res., 11,* 346—351 and Glock, G. E. and Mcclean, P. (1953). Further studies on the properties of glucose-6-phosphate dehydrogenase and 6-phosphogluconate dehydrogenase of rat liver, *Biochem. J., 55,* 400—408. Values are mean ± SD for three separate determinations, with pooled epidermal tissue from 4 mice used for each determination. The mean weight of the food restricted mice was 18.4 ± 0.8 g, n=12. The average food consumed was 2.4 g/mouse/day for food restricted and 3.9 g/mouse/day for the *ad libitum* fed mice.

TABLE 6

Effect of Orally Administered DHEA or 19 on TPA Stimulation of $^3$H-Thymidine Incorporation in Mouse Epidermis

| Treatment | Specific Activity (cpm/μg DNA) |
|---|---|
| No steroid | 58.1 ± 6.7 (n = 3) |
| No steroid plus TPA | 155 ± 13.8 (n = 3) |
| TPA plus DHEA (400 mg/kg p.o.) | 59.5 ± 5.4 (n = 2) |
| TPA plus DHEA (200 mg/kg p.o.) | 118 ± 4.5 (n = 3) |

Male ICR mice were orally intubated with steroid suspended in sesame oil (0.5 ml/mouse) at the indicated dose. Mice not receiving steroid were given sesame oil alone. One hour later mice received topical application of TPA and 20 hours later the rate of $^3$H-thymidine incorporation into the epidermis was determined as described in Table 3.

TABLE 7

Effect of Orally Administered DHEA, or two weeks of Food Restriction on [$^3$H]DMBA Binding to Skin DNA

| Treatment | Specific Activity (cpm/µg DNA) |
|---|---|
| No steroid | 65.7 ± 9.6 (n = 3) |
| DHEA (400 mg/kg, p.o.) | 73.1 ± 16.5 (n = 3) |
| No steroid, Food restricted | 32.3 ± 3.5 (n = 3) |

Male A/J mice were orally intubated with steroid suspended in sesame oil (0.5 ml/mouse) at the indicated dose. Mice not receiving steroid were given sesame oil alone. Food restricted mice received approximately 60% of food of *ad libitum* fed for two weeks. One hour after oral intubation, topical [$^3$H]DMBA was applied to the skin, and the amount bound to DNA was determined 12 hours later as described in Table 3.

TABLE 8

Effect of Orally Administered 20 on TPA Stimulation of $^3$H-Thymidine Incorporation in Mouse Epidermis

| Treatment | Specific Activity (cpm/µg DNA) |
|---|---|
| TPA plus 20 (150 mg/kg, p.o.) | 11 ± 2.2 (n = 3) |
| TPA plus 20 (100 mg/kg, p.o.) | 13.6 ± 2.4 (n = 3) |
| TPA plus 20 (50 mg/kg, p.o.) | 16.3 ± 1.6 (n = 3) |

Male ICR mice were orally intubated with steroid suspended in sesame oil (0.5 ml/mouse) at the indicated dose. Mice not receiving steroid were given sesame oil alone. One hour later mice received topical applications of TPA, and 20 hours later the rate of $^3$5H-thymidine incorporation into the epidermis was determined as described in Table 3.

TABLE 9

Effect of Orally Administered DHEA or 20 on [$^3$H]DMBA Binding to Skin DNA

| Treatment | Specific Activity (cpm/µg DNA) |
|---|---|
| No steroid | 142 ± 5.9 (n = 3) |
| DHEA (400 mg/kg, p.o.) | 92 ± 17.6 (n = 3) |
| 20 (200 mg/kg, p.o.) | 43.7 ± 1.5 (n = 3) |
| 20 (100 mg/kg, p.o.) | 48.4 ± 2.6 (n = 3) |

Male A/J mice were orally intubated with steroid suspended in sesame oil (0.5 ml/mouse) at the indicated dose. Mice not receiving steroid were given sesame oil alone. One hour after oral intubation, topical [$^3$H]DMBA was applied to the skin, and the amount bound to DNA was determined 12 hours later as described in Table 3.

Anti-Autoimmune Activity

New Zealand Black (NZB) mice develop a progressive autoimmune, Coomb's positive hemolytic anemia with age. It has been previously found that long-term treatment of NZB mice with DHEA significantly inhibits the rate of development of the autoimmune anemia. There is a reasonable probability that steroids of the present invention will also retain the anti-autoimmune activity of DHEA.

The compound 3β-methyl-5-androsten-17-one (referred to as DE-7) was synthesized to overcome the estrogenic and possible androgenic effects of DHEA. DHEA injected at 60 mg/kg s.c. for 3 days into sexually immature rats produces uterine enlargement as a consequence of DHEA metabolism into estrogens (Knudsen, T. T. and Makesh, V. B., 1975. Initiation of precocious sexual maturation in the immature rat treated with dehydroepiandrosterone, Endocrinology *97*, 458). On the contrary, injection of DE-7 results in a reduction in uterine weight.

Experimental Data

Female CD rats, 26—27 days old, were used. Rats were injected subcutaneously for 3 days with either DHEA of DE-7 in propylene glycol. Control rats received propylene glycol alone. The rats were killed on the 4th day and the uteri were removed and weighed.

| Group | Mean Uterine Weight ± S.D. (mg/100 g body weight) |
|---|---|
| Control | 195 ± 24 (n = 6) |
| DHEA (60 mg/kg) | 222 ± 77 (n = 6) |
| DE-7 (60 mg/kg) | 126 ± 87 (n = 6) |
| DE-7 (120 mg/kg) | 111 ± 27 (n = 6) |

Thus DE-7, when injected s.c. at a dose of 60 mg/kg or higher, apparently acts as an anti-estrogen.

Anti-Androgenic Effect of DE-7

In addition to the anti-estrogenic action of DE-7, this steroid also has anti-androgenic properties. Treatment of male A/J mice with DE-7 for 4 weeks significantly reduced the weight of the seminal vesicles and prostate glands.

Experimental Data

Male A/J mice (5 weeks old) were obtained from the Jackson Laboratory and were housed in polycarbonate cages (5 mice/cage) in animal quarters maintained at 24 ± 1°C with 12 hours of light and 12 hours of darkness each day. One week after arrival, mice were placed on a chow diet containing either 0.18% or 0.09% DE-7 or without steroid. Mice were weighed weekly. After 4 weeks, the mice were killed, and the seminal vesicles plus prostate glands were dissected out and weighed.

| Group | Mean Seminal Vesicle plus Prostate Weight ± S.D. (mg/100 g body weight) |
|---|---|
| Control | 5.5 ± 0.9 (n = 6) |
| DE-7 (0.09%) | 4.0 ± 0.7 (n = 6) |
| DE-7 (0.18%) | 3.4 ± 0.11 (n = 6) |

Both the anti-estrogenic and anti-androgenic activity of DE-7 would very likely make it unacceptable as a drug for humans.

Use of 16-Substituted Derivatives of DE-7 to Overcome Anti-Estrogenic and Anti-Androgenic Activities

Because of the structural similarity between DE-7 and DHEA, it seemed reasonable that DE-7 might competitively inhibit the conversion of DHEA to 5-androsten-3,17-dione by the enzyme 3-β-hydroxysteroid

dehydrogenase. 5-Androsten-3,17-dione is a precursor to both testosterone and estrone. Thus DE-7 may competitively inhibit the conversion of endogenous DHEA into testosterone and estrone, and this may account for both the anti-estrogenic and anti-androgenic activity of this steroid.

In the paper entitled "Inhibitions of Human Placental $C_{10}$ and $C_{21}$ 3β-Hydroxysteroid Dehydrogenases" by A. S. Goldman and K. Sheth in *Biochimica Biophysica Acta 315,* 253 (1973) a series of steroids were tested for their capacity to inhibit 3β-hydroxysteroid dehydrogenase. It was noted that either a *16α-OH* or *16 oxime* substitution in the $\Delta^5$ androstene series or a *6β OH* substitution in the $\Delta^4$ androstene series greatly reduced the capacity of the steroid to inhibit the enzymatic conversion of DHEA into estrogens. Accordingly in order to overcome the anti-estrogenic and anti-androgenic activity of DE-7, the following steroids are proposed.

Activity of 3β-Methyl-16-oxime-5-androsten-17-one

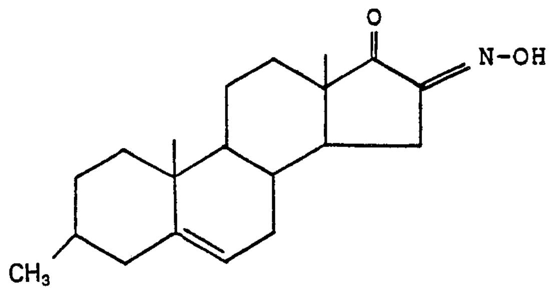

3β-Methyl-16-oxime-5-androsten-17-one (DE-7-16 oxime)

a. Activity in estrogen — anti-estrogen test

Female CD rats, 26—27 days old, were injected subcutaneously for 3 days with either DE-7 or DE-7-16α-methyl at 60 mg/kg in propylene glycol. Controls received propylene glycol alone. On the 4th day the rats were killed and the uteri were dissected out and weighed.

| Control | Mean uterine weight ± S.D. 1 mg/100 g body weight |
|---|---|
| Control | 1.97 ± 0.20 (n = 7) |
| DE-7 | 1.15 ± 0.16 (n = 7) |
| DE-7-16-oxime | 1.99 ± 0.24 (n = 7) |

DE-7-16-oxime shows no apparent estrogenic nor anti-estrogenic activity. DE-7 shows highly significant anti-estrogenic action.

b. Anti-obesity Action

Male A/J mice (5 weeks old) were obtained from the Jackson Laboratory and were housed in polycarbonate cages (5 mice/cage) in animal quarters maintained at 24 ± 1°C with 12 hours of light and 12 hours of darkness each day. One week after arrival, the mice were placed on a show diet containing DHEA, and DE-7-16-oxime at a dose of 0.54%. This compound produced no demonstratable anti-obesity effect at a dose which is about twice what is needed with DHEA to produce an anti-obesity effect.

The compounds, i.e. therapeutic agents of this invention, may be administered alone or in combination with pharmaceutically-acceptable carriers, the proportion of which is determined by the solubility and chemical nature of the compound, chosen route of administration and standard pharmaceutical practice. For example, they may be administered orally in the form of tablets, pills or capsules containing such excipients as starch, milk sugar, certain types of clay and so forth. They may be administered orally in the form of solutions which may contain coloring and flavoring agents or they may be injected parenterally, that is, intramuscularly, intravenously or subcutaneously. For parenteral administration, they may be used in the form of a sterile solution containing other solutes, for example, enough saline or glucose to make the solution isotonic.

The physician will determine the dosage of the present therapeutic agents which will be most suitable and it will vary with the form of administration and the particular compound chosen, and furthermore, it will vary with the particular patient under treatment. He will generally wish to initiate treatment with small dosages substantially less than the optimum dose of the compound and increase the dosage by small increments until the optimum effect under the circumstances is reached. It will generally be found that when the composition is administered orally, larger quantities of the active agent will be required to

produce the same effect as a smaller quantity given parenterally. The compounds are useful in the same manner as comparable therapeutic agents and the dosage level is of the same order of magnitude as is generally employed with these other therapeutic agents.

When given orally, the therapeutic doses of the compounds of the present invention are generally in the range of from about 4 to about 450 mg/kg/day depending upon the particular mammalian host and the particular effect desired, e.g. cancer preventive, anti-obesity, anti-diabetes, when given parenterally, the compounds are administered generally in dosages of, for example, 0.5 to about 15 mg/kg/day, also depending upon the host and effect desired.

## Claims

1. A compound of the formula:

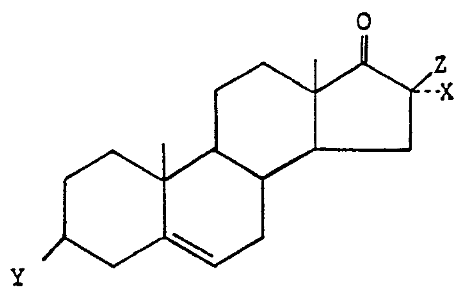

wherein

Y is $C_{1-5}$ alkyl; and

X and Z are independently hydrogen, halogen $C_{1-5}$ alkyl or hydroxy, or X and Z taken together are hydroxyimino with the proviso that when Y is methyl and Z is hydrogen, X is other than methyl or hydroxy and with the further proviso that X and Z are not both hydrogen and that said compound is not 3β-methyl-16α-ethyl-5-androsten-17-one or 16α-bromo-3β-methyl-5-androsten-17-one.

2. The compound according to Claim 1 having the formula:

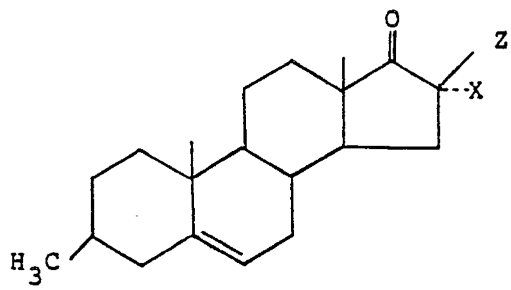

wherein X is halogen, or $C_{1-5}$ alkyl,

Z is hydrogen or $C_{1-5}$ alkyl and

X and Z taken together are hydroxyimino.

3. The compound according to Claim 2 wherein said alkyl is methyl.

4. The compound according to any of Claims 1—3 wherein said halogen is bromine or fluorine.

5. The compound according to any of Claims 1—4 wherein said halogen is fluorine.

6. The compound according to Claims 1 or 2 which has the formula:

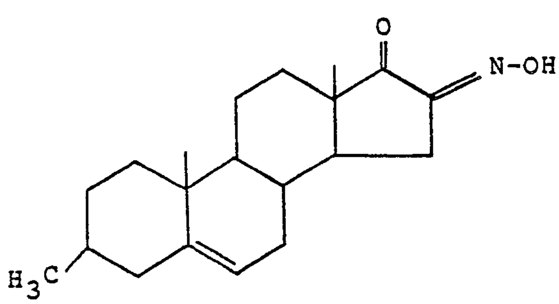

7. The compound according to Claims 1 or 2 which has the formula:

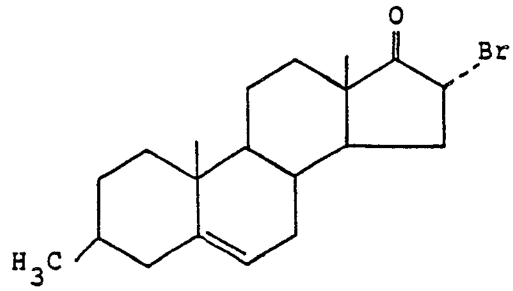

8. The compound according to Claims 1 or 2 which has the formula:

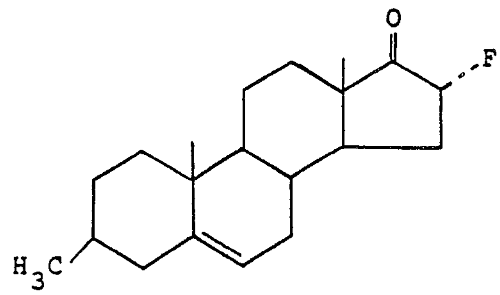

9. The compound according to Claims 1 or 2 which has the formula:

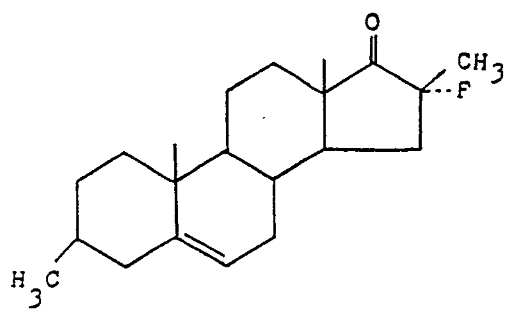

10. A therapeutic composition comprising a compound according to any of Claims 1—9 and a pharmaceutical carrier therefor.

11. Use of a compound according to any of Claims 1 to 9 for the preparation of a therapeutic anti-cancer, anti-obesity, anti-hyperglycemic, anti-autoimmune and anti-hypercholesterolemic composition.

**Patentansprüche**

1. Eine Verbindung der Formel

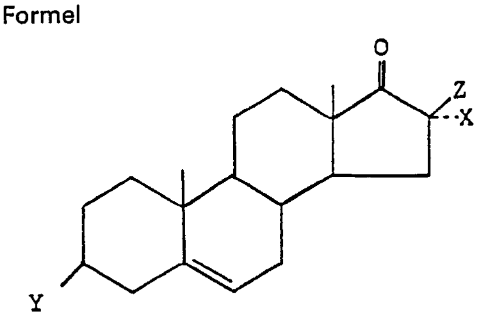

worin bedeuten

Y $C_{1-6}$-Alkyl und

X und Z unabhängig voneinander Wasserstoff, Halogen, $C_{1-5}$-Alkyl oder Hydroxy, oder X und Z zusammen Hydroxyimino mit dem Proviso, dass, wenn Y Methyl und Z Wasserstoff ist, X kein Methyl oder Hydroxy ist, und mit dem weiteren Proviso, dass X und Z nicht beide Wasserstoff sind und dass die Verbindung nicht 3β-Methyl-16 alpha-ethyl-5-androsten-17-on oder 16 alpha-Brom-3β-methyl-5-androsten-17-on ist.

2. Verbindung gemäss Anspruch 1, mit der Formel

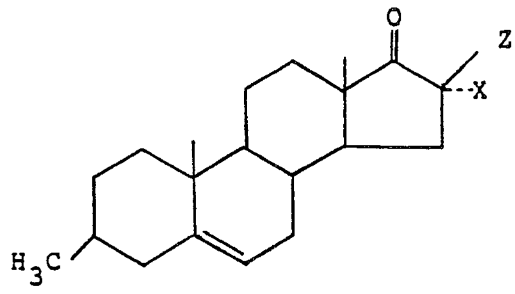

worin X Halogen oder $C_{1-5}$-Alkyl ist,
Z Wasserstoff oder $C_{1-5}$-Alkyl ist und X und Z zusammen Hydroxyimino sind.

3. Verbindung gemäss Anspruch 2, worin das Alkyl Methyl ist.

4. Verbindung gemäss einem der Ansprüche 1 bis 3, worin das Halogen Brom oder Fluor ist.

5. Verbindung gemäss einem der Ansprüche 1 bis 4, in welcher das Halogen Fluor ist.

6. Verbindung gemäss Ansprüchen 1 oder 2 der Formel

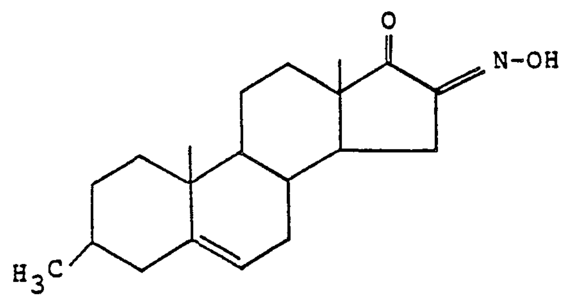

7. Verbindung gemäss Ansprüchen 1 oder 2 der Formel

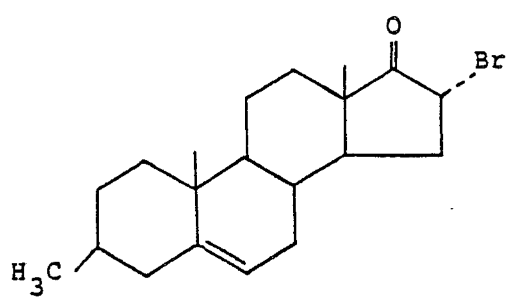

8. Verbindung gemäss Ansprüchen 1 oder 2 der Formel

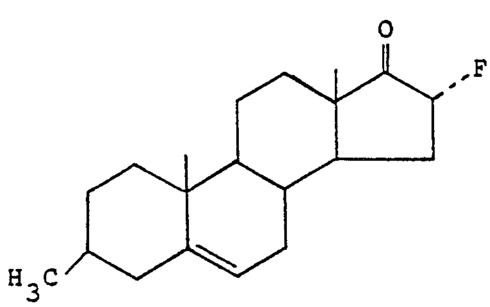

9, Verbindung gemäss Ansprüchen 1 oder 2 der Formel

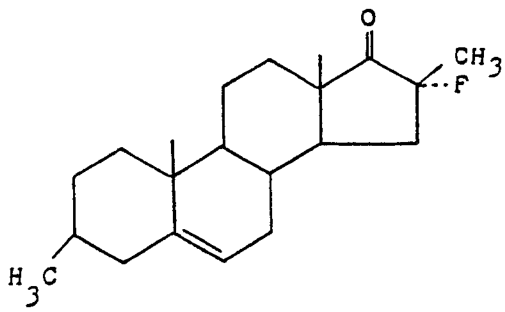

10. Therapeutische Zusammensetzung, enthaltend eine Verbindung gemäss einem der Ansprüche 1 bis 9 und einen pharmazeutischen Träger dafür.

11. Verwendung einer Verbindung gemäss einem der Ansprüche 1 bis 9 zur Herstellung einer therapeutischen Verbindung gegen Krebs, Fettsucht, Hyperglykämie, Autoimmunität und Hypercholesterolämie.

**Revendications**

1. Composé de formule:

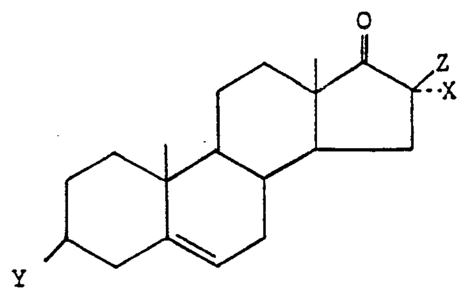

dans laquelle:

Y représente alkyle en $C_{1-5}$; et

X et Z représentent indépendamment hydrogène, halogène, alkyle en $C_{1-5}$ ou hydroxy, ou bien X et Z, pris ensemble, représentent hydroxyimino, avec la condition que, lorsque Y représente méthyle et Z représente hydrogène, X est autre que méthyle ou hydroxy, et avec la condition supplémentaire que X et Z ne représentent pas simultanément hydrogène et que ledit composé ne soit pas la méthyl-3β éthyl-16α androstène-5 one-17 ou la bromo-16α méthyl-3β androstène-5 one-17.

2. Composé selon la revendication 1, représenté par la formule:

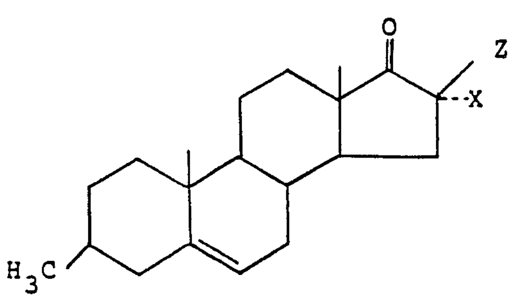

dans laquelle:

X représente halogène ou alkyle en $C_{1-5}$;

Z représente hydrogène ou alkyle en $C_{1-5}$ et

X et Z, pris ensemble, représentent hydroxyimino.

3. Composé selon la revendication 2, dans lequel ledit alkyle représente méthyle.

4. Composé selon l'une des revendications 1 à 3, dans lequel ledit halogène représente brome ou fluor.

5. Composé selon l'une des revendications 1—4, dans lequel ledit halogène représente le fluor.

6. Composé selon l'une des revendications 1 et 2, qui a pour formule:

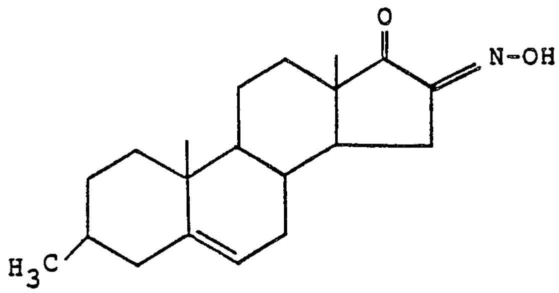

7. Composé selon l'une des revendications 1 et 2, qui a pour formule:

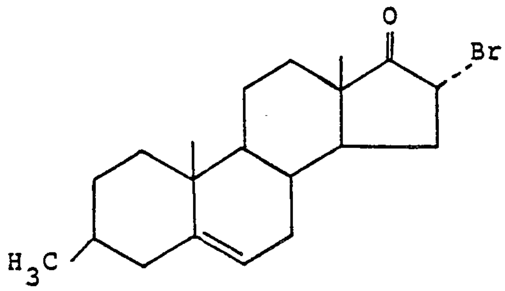

8. Composé selon l'une des revendications 1 et 2, qui a pour formule:

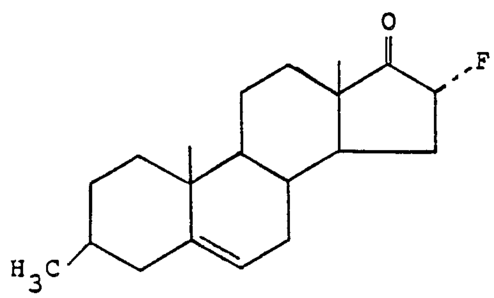

9. Composé selon l'une des revendications 1 et 2, qui a pour formule:

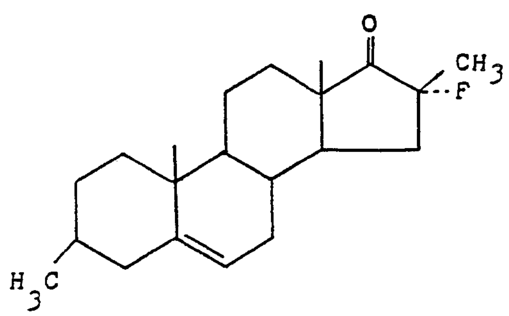

10. Composition thérapeutique comprenant un composé selon l'une des revendications 1—9 et un support pharmaceutique pour celui-ci.

11. Utilisation d'un composé selon l'une des revendications 1 à 9 pour la préparation d'une composition thérapeutique anti-cancer, anti-obésité, anti-hyperglycémique, anti-autoimmune et anti-hypercholestérolémique.